# EUROPEAN PATENT APPLICATION

(11) **EP 4 451 178 A1**
(43) Date of publication of application: **23.10.2024**
(21) Application number: 24171016.9
(22) Date of filing: 18.04.2024
(51) Int. Cl.: G06N 3/091, G16H 50/20

(54) **OPERATIONALISING FEEDBACK LOOPS**

(30) Priority: 18.04.2023 US 202363496798 P
(71) Applicant: Palantir Technologies Inc., Denver, CO 80202 (US)
(72) Inventor: EDWARDS, Alexander, London (GB); MUKHERJEE, Anirvan, Brooklyn (US); KEBUDI, David, New York (US); ARORA, Megha, London (GB); KRISHNAN, Sriram, Jersey City (US); SCHRADER, Max, Germering (DE); WINSSINGER, Jessica, New York (US); HOEFER, Philipp, Munich (DE)
(74) Representative: Derry, Paul Stefan

(57) **Abstract**

Disclosed herein is a method of providing feedback to a machine learning model. The method includes allowing a user to observe an output of a trained machine learning model; allowing the user to input feedback to the machine learning model based on the output, wherein the feedback is on at least one of a model level or on a training dataset level; and incorporating the feedback into the machine learning model to improve the machine learning model, wherein the method is performed using one or more processors. Disclosed herein are one or more computer-readable storage media including computer executable instructions which when executed by the one or more processors cause the one or more processors to perform the computer-implemented method. Disclosed herein is a computer system which includes one or more processors and one or more computer-readable storage media which include computer executable instructions which when executed by the one or more processors cause the one or more processors to perform the computer-implemented method.

## Description

### Field of the Disclosure

This disclosure relates to feedback loops, and more particularly how feedback loops are used to improve machine learning models.

### Background

Recently, organizations have been leveraging artificial intelligence (AI), machine learning (ML), and optimization (OPT) models to improve organizational decision-making. Many model-building tools allow for building, training, and tuning models, as well as utilize out-of-the-box models to fit against specific datasets. However, the journey from development sandbox to impactful operational tool for users remains precarious. One-off AI/ML/OPT models may be developed to address specific business needs via a point-solution or made available on an API endpoint, but many organizations lack the overarching framework to ensure that data, models, and decisions can be captured and deployed across use cases - resulting in fragmentation and limited learning. Another problem is to capture and integrate the expert know-how that exists in the heads of experts in the field. The present disclosure shows technical approaches to address these problems. Furthermore, the approaches disclosed therein allow machine learning models to continuously learn and improve.

### Summary

According to a first aspect, a method is provided that includes providing feedback to a machine learning model. The method includes allowing a user to observe an output of a trained machine learning model; allowing the user to input feedback to the machine learning model based on the output, wherein the feedback is on at least one of a model level or on a training dataset level; and incorporating the feedback into the machine learning model to improve the machine learning model, wherein the method is performed using one or more processors.

According to a second aspect, one or mor computer-readable storage media are provided which include computer executable instructions which when executed by one or more processors cause the one or more processors to perform a method which includes providing feedback to a machine learning model. The method includes allowing a user to observe an output of a trained machine learning model; allowing the user to input feedback to the machine learning model based on the output, wherein the feedback is on at least one of a model level or on a training dataset level; and incorporating the feedback into the machine learning model to improve the machine learning model, wherein the method is performed using one or more processors.

According to a third aspect, a computer system is provided. The computer system includes one or more processors; and one or more computer-readable storage media comprising computer executable instructions which when executed by the one or more processors cause the one or more processors to perform a method which includes providing feedback to a machine learning model. The method includes allowing a user to observe an output of a trained machine learning model; allowing the user to input feedback to the machine learning model based on the output, wherein the feedback is on at least one of a model level or on a training dataset level; and incorporating the feedback into the machine learning model to improve the machine learning model, wherein the method is performed using one or more processors.

Advantages that may be experienced include that model feedback can be used to monitor, re-train and improve models. As end users actions are taken in operational contexts, decisions are captured back into an ontology, and made seamlessly available for model monitoring, as well as to labelling and training environments. This feedback loop can help to ensure that data scientists are able to quickly evolve models to meet ever-changing real-world conditions.

These and other features of the systems, methods, and non-transitory computer readable media disclosed herein, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for purposes of illustration and description only and are not intended as a definition of the limits of the scope of protection.

### Brief Description of the Drawings

Certain features of various embodiments of the present technology are set forth with particularity in the appended claims. A better understanding of the features and advantages of the technology will be obtained by reference to the following detailed description that sets forth illustrative embodiment, in which the principles are utilized, and the accompanying drawings of which:
Fig. 1 shows a flowchart of a method of providing feedback to a machine learning model according to embodiments of this disclosure.
Fig. 2 shows a flowchart of a feedback loop in which quantitative feedback is incorporated into the machine learning model according to embodiments of this disclosure;
Fig. 3 illustrates a flowchart of a feedback loop in which qualitative feedback is manually incorporated into the machine learning model according to embodiments of this disclosure;
Fig. 4 shows a flowchart of a feedback loop in which quantitative feedback is given to improve a prognostic lung cancer model according to embodiments of this disclosure;
Fig. 5 is a flowchart that illustrates the use of feedback loops in which quantitative feedback is given to improve a machine learning model that is able to determine whether to accept or not a potential client by a financial institute.
Fig. 6 is a hardware architecture in which embodiments of this disclosure are implemented.

The figures depict various embodiments of the disclosed technology for purposes of illustration only. One skilled in the art will readily recognize from the following discussion that alternative embodiments of the structures and methods illustrated in the figures can be employed without departing from the principles of the disclosed technology described herein.

### Detailed Description of Embodiments

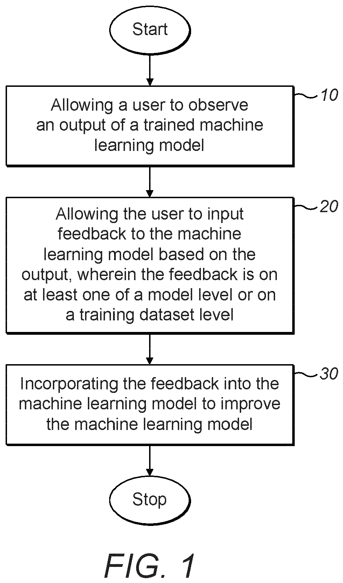

Fig. 1 shows a flowchart of a method of providing feedback to a machine learning model. However, before discussing the embodiment in Fig. 1 in more detail, a few items of this disclosure will be discussed.

Some of the embodiments relate to a method of providing feedback to a machine learning model. The method includes allowing a user to observe an output of a trained machine learning model; allowing the user to input feedback to the machine learning model based on the output, wherein the feedback is on at least one of a model level or on a training dataset level; and incorporating the feedback into the machine learning model to improve the machine learning model. The method is performed using one or more processors.

Feedback loops allow connecting model development and management lifecycle with the operational "response" from the real world (source systems) or operational end users- allowing the model to continuously learn and improve. Feedback brings external information into the machine learning system. This is possible in Foundry in a high fidelity way due to Palantir ontology writeback functionality. Feedback loop solution within Foundry allows for bias monitoring of the feedback, as well as the bias monitoring of the source, creating the rail guards around training the most successful model.

In some of the embodiments, the feedback on the model level includes qualitative feedback on the machine learning model. In some of these embodiments, the feedback on the model relates to features and/or hyperparameters of the machine learning model. The machine learning model is re-trained and it is looped back to the step of allowing a user to observe an output of a trained machine learning mode to iteratively improve the machine learning model. In some of these embodiments, the user gives feedback on the features and/or hyperparameters based on the current performance of a model. The term "qualitative feedback" means, for example, that the user when looking at the prediction output gives general feedback that the machine learning model does not give a good performance or that one or more variables should be ignored. This qualitative feedback requires retraining of the machine learning model.

In some of the embodiments, the feedback on the dataset level includes quantitative feedback on the output of the machine learning model and wherein incorporating the feedback into the machine learning model includes writing back the feedback in a write-back dataset; and merging the write-back dataset with the training dataset. The method further includes retraining the machine learning model with the training dataset; and looping back to the step of allowing a user to observe an output of a trained machine learning model to iteratively improve the machine learning model.

In some of the embodiments, the user is allowed to flag the prediction output of the machine learning model as a false positive for a binary classification problem.

In some of the embodiments, when the prediction output is a predicted time period for an event the user is allowed to indicate how long the event took in reality.

In some of the embodiments, the event is a maintenance task. In the embodiments of a predictive maintenance task, the user gives feedback to the underlying machine learning model as to how long the actual maintenance task took. This allows for iteratively retraining the machine learning model.

In some of the embodiments, the incorporating of the feedback is performed as write-backs. In some of the embodiments, the feedback is written in a write-back dataset and the write-back dataset is merged with the training dataset. This modified training dataset is used to re-train the model.

In some of the embodiments, the qualitative feedback is reviewed by a user and manually incorporated. Qualitative feedback is feedback that normally cannot be directly incorporated into the machine learning model. For instance, qualitative feedback is a statement by a user that "the model does not perform well" or "the model does not give an accurate prediction in the present use case". In these cases, a machine learning expert, i.e. model builder has to modify the machine learning model, e.g. for example change some hyperparameters or remove features from the model.

In some of the embodiments, the quantitative feedback is automatically incorporated in a modelling pipeline to retrain the machine learning model with better data. Quantitative feedback is, for example, predicted data that the user flags as false positive or the duration that the actual maintenance task took in reality. This information can be written into write-back datasets that are merged with the training dataset. The modified training dataset is used to retrain the machine learning model.

In some of the embodiments, the feedback captures accumulated experience and know-how of subject-matter experts over time.

In some of the embodiments, the feedback is bias monitored.

In some of the embodiments, the training dataset is bias monitored.

In some of the embodiments, the method of providing feedback (feedback loop) has a high latency, while in other embodiments the method has a low latency. Latency, from a general point of view, is a time delay between the cause and the effect of some physical change in the system being observed.

In some of the embodiments, the feedback on the model level and the feedback on the dataset level are input to the machine learning model via one common graphical user interface. This allows for that different training datasets do not have to be sent to different domain experts. Also machine learning model experts can input and review input given by domain experts.

In some of the embodiments, the method is used in a system to predict a variable based on medical images. The user is a clinician and allowing the user to input feedback comprises selecting an area of the image by the clinician, passing the selected area of the image to the machine learning model for a preliminary prognosis, evaluating the output of the model by the clinician and inputting the evaluation as feedback. In some of the embodiments, the medical images show images of a human organ.

In some of the embodiments, the feedback is quantitative feedback and includes medical images annotated by the clinician.

In some of the embodiments, the variable to be predicted is a survival rate of lung cancer patients and the medical images are images showing human lungs or parts thereof. Predicting survival rates in lung cancer patients depends on a number of different factors such as the age and underlying health of the person. It is a complex process that relies on clinical expertise and increasingly incorporating statistical models. Some of the embodiments relate to developing and deploying these models that more accurately predict survival rates based on the data presented and combining feedback from subject matter experts in a real-time setting. This helps clinician in managing their patients more holistically with next best treatment options.

In some of the embodiments, the method is used in a system to assess whether or not to accept new clients of a financial institute in view of sanctions.

Some of the embodiments relate to one or mor computer-readable storage media including computer executable instructions which when executed by one or more processors cause the one or more processors to perform the method of any one of the preceding claims.

In some of the embodiments, the method of providing feedback to a machine learning model is integrated into a larger data analytics system (such as Palantir's proprietary system "Foundry") which includes an ontology which is a shared source of truth for decision-making and decision capture across a large organization. This advanced operationalization helps to close the loop between AI/ML/OPT and operations in several ways:

In some of the embodiments, the system is able to capture decisions. As end users make decisions, the system captures them back in the ontology - in accordance with governance paradigms - and with a full lineage trail that encompasses both data and model inputs.

In some of the embodiments, the system allows for automatically writing-back to systems of action. With the data analytics suite of bidirectional connectors, all decisions made by end users are recorded and written back to both the ontology and systems of action. This orchestration means that organizations can benefit from the data analytics system's operational AI/ML/OPT without needing to supplant any core operational systems. The system maintains a complete log of decisions and states to ensure auditability and complete transparency. Decisions are non-destructive since underlying data is brought in through system integrations and versioned.

In some of the embodiments, the system may use model feedback to monitor, re-train and improve models. As end user actions are taken in operational contexts, the decisions are captured back into the ontology, and made seamlessly available for model monitoring, as well as to labeling and training environments. This feedback loop ensures that data scientists are able to quickly evolve models to meet ever-changing real-world conditions.

Returning now to Fig. 1 which shows a method of providing feedback to a machine learning model. At 10, the method includes allowing a user to observe an output of a trained machine learning model. At 20, the user is allowed to input feedback to the machine learning model based on the output, wherein the feedback is on at least one of a model level or on a training dataset level. At 30, the feedback is incorporated into the machine learning model to improve the machine learning model. The application of the method shown in Fig. 1 will be illustrated in more detail by means of three different use cases.

### 1. Use case: Predictive Maintenance Tool

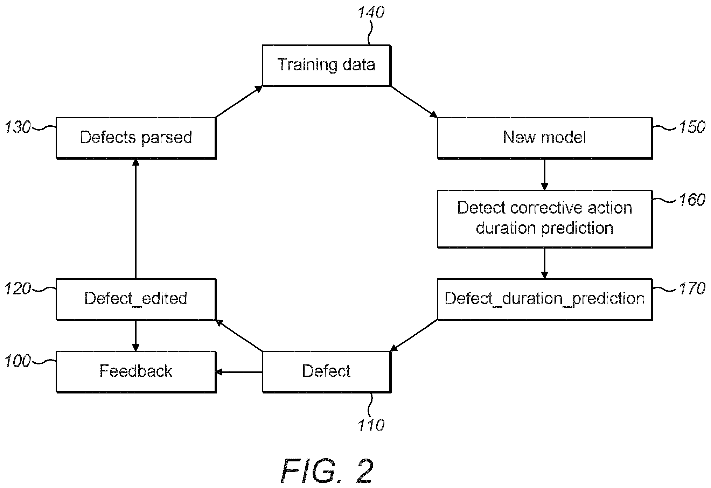

Fig. 2 shows a predictive maintenance and task management workflow based on quantitative feedback (direct feedback) that is automatically incorporated into the machine learning model. Direct feedback is when the feedback we are getting from the user is the target variable which can be used to create an updated dataset.

The drawing shows how feedback loops are employed in order to improve machine learning models and improve the learning. The system shown is a predictive maintenance task system and the feedback received by the user is quantitative feedback / direct feedback. The system predicts how long it will take until a system becomes operational again after it has failed. In the present example, a maintenance task / defect gets reported by a plane pilot which creates a task object. The task object has a "predicted_labour_hours" column, which gets populated by running a batch deployment on an hourly cadence. After the predicted labour hours for a maintenance task is displayed, at 100, the user can input feedback into the system. In the present example, at 110, the user detects a defect for which the time predicted by the system for the maintenance task substantially differs from the time the maintenance task actually took. Via a graphical user interface, the user is allowed to input feedback on how long the maintenance task actually took. At 120, the actual time duration of the maintenance task is written back into a writeback dataset. At 130, all the defects are parsed and are merged with the training dataset. At 140, a modified training dataset is obtained which is used to train the machine learning model. The machine learning model gets re-trained every night with new data and batch deployment on reported tasks happen on an hourly schedule. In this example, only the training dataset is modified but not parameters / hyperparameters of the model itself. Hence, in this example only quantitative feedback is provided but not qualitative feedback. By training the machine learning model with new training data, a new model is obtained at 150. At 160, a modelling objective "Defect Corrective Action Duration Prediction" is used. At 170, the system receives input and predicts the duration of the corrective action using the new machine learning model. Thereby, the new/improved machine learning model is evaluated. Should the pilot again see a predicted time that does not appear to be correct (or if he inputs a defect that has already occurred and he therefore certainly knows the time of the corresponding maintenance task (evaluation data)), he can input his feedback by correcting the prediction time. It should be mentioned that in some of the embodiments the person who inputs the data is controlled for individual bias. Furthermore, new data inevitably introduces bias and potentially drag. There is a need to collect prediction/actual value pairs to track their impact on the model's performance and thereby the effectiveness of the feedback loop is assessed.

The present embodiment shows model monitoring which is often considered to be a part of the model evaluation phase. During evaluation, the question the model owner is trying to answer is: Is the model performant enough on evaluation data? Evaluation data typically contains ground truth information (as known as labels). When trying to monitor a deployed model, it can no longer be assumed that ground truth information is available. The type of questions model monitoring aims to answer include:
1. Does the model input seem reasonable? - Looking at high-level distributions for model features and understanding if they are still comparable with the distributions in the data that was used to train the model.
2. Does the model output seem reasonable? - Looking at model prediction distribution and understanding if it is/they are comparable with the distributions in the outputs at training time.

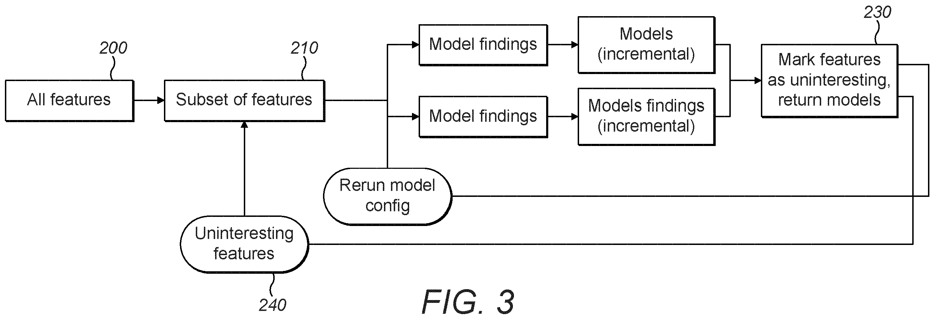

Fig. 3 shows a flowchart that indicates how qualitative feedback (indirect feedback) is manually incorporated into the machine learning model. Indirect feedback is when the feedback that is received from the user is not the target variable which can be used to directly re-train the underlying predictive model. It should be mentioned that qualitative feedback is reviewed by someone and can inform change in the model methodology itself. In this example, the aim is to identify which features could affect a specific target. Models are run that would output features highly correlated with the target. Many of those features were causal but in the wrong direction i.e. caused by the target, not causing the target. Those features would account for most of the variance of the model, so needed to be excluded. Machine learning experts are allowed to give qualitative feedback to exclude certain features from the model. At 200, all features of the machine learning model are initially considered. At 210, a machine learning expert selects a subset of all features. The model is run with the subset of the features. At 210, the machine learning experts could select findings from a model and mark the underlying features as "uninteresting" at 230. The uninteresting features 240 are removed from the subset of features 210. The target is defined and model type to be rerun is defined by the machine learning expert. The uninteresting features 240 are removed from the subset of features 210 and the model is rerun with the reduced subset of features.

### 2. Use case: Predicting survival rates in lung cancer patients

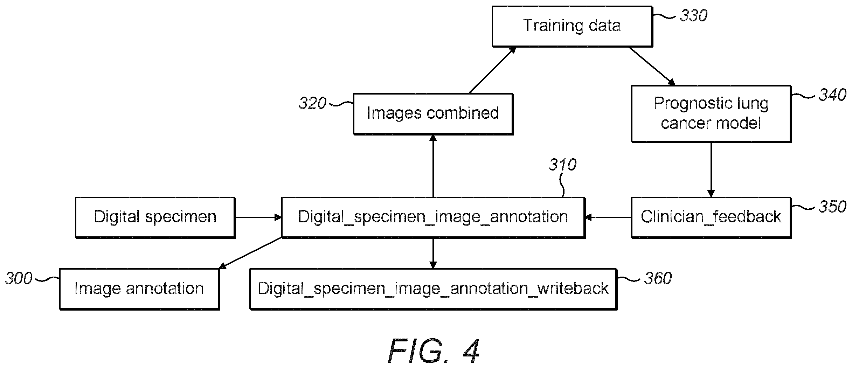

Fig. 4 shows a flowchart of a feedback loop with the aim to improve a prognostic lung cancer model. At 300, the clinician has access to an image annotation application, such as a point-and-click application tool. The clinician selects an area of the lung image that looks important to them and pass that to a prognostic lung cancer model 340 for a preliminary prognosis. The model 340 outputs a predicted survival rate. If the model output is not as they would have expected, clinician can flag it (qualitative feedback) for review by the model owner, allowing data science teams to understand when and why their model is underperforming. More importantly, if the clinician is of the opinion that this survival rate is too high, he can give quantitative feedback and label model output as incorrect. He can annotate the image at 310 by indicating a survival rate that appears to be more correct to him. At 360, the correct annotation is written back to an annotated labelled dataset. At 320, several annotates images are combined and at 330, they are merged with the existing model training data. Thereby, the training data is improved and using the improved model training data for training of the prognostic lung cancer model 340 will lead to an improved lung cancer model that will predict survival rates of lung cancer patients more realistically. Allowing clinicians to give real-life feedback accelerates the process of retraining and redeploying models from months to days.

### 3. Use case: Enhanced Sanctions Screening

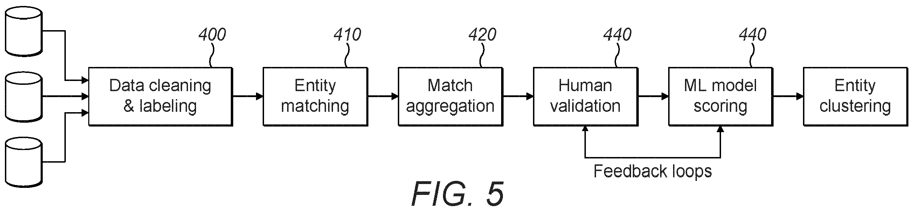

Fig. 5 shows a flowchart in which a feedback loop is used to improve a machine learning model that is able to screen potential customers of a financial institute against a PEP and Sanctions Lists (as part of the Know Your Customer (KYC) stage). Historically, analysts have needed to devote significant time to manual searches of potential customers to ensure they adhere to bank-specific requirements and do not have ties to entities on global common sanctions lists. These costly manual searches often mean less time for analysis, resulting in lower accuracy and more consistent regulatory fines. At 400, required data from the bank's potential future customers is cleaned. At 410 the data is run through entity matching and match aggregation, and is given a risk score from the model. At 430, during "human validation", analysts validate the score of the model by confirming or rejecting final decisions. Thereby, quantitative feedback is given which is merged with a training dataset that is used to update / re-train the machine learning model.

### Hardware Implementation

The techniques described herein are implemented by one or more special-purpose computing devices. The special-purpose computing devices may be hard-wired to perform the techniques, or may include circuitry or digital electronic devices such as one or more application-specific integrated circuits (ASICs) or field-programmable gate arrays (FPGAs) that are persistently programmed to perform the techniques, or may include one or more hardware processors programmed to perform the techniques pursuant to program instructions in firmware, memory, other storage, or a combination thereof. Such special-purpose computing devices may also combine custom hard-wired logic, ASICs, or FPGAs with custom programming to accomplish the techniques. The special-purpose computing devices may be desktop computer systems, server computer systems, portable computer systems, handheld devices, networking devices or any other device or combination of devices that incorporate hard-wired and/or program logic to implement the techniques.

Computing device(s) are generally controlled and coordinated by operating system software, such as iOS, Android, Chrome OS, Windows XP, Windows Vista, Windows 7, Windows 8, Windows Server, Windows CE, Unix, Linux, SunOS, Solaris, iOS, Blackberry OS, VxWorks, or other compatible operating system. In other embodiments, the computing device may be controlled by a proprietary operating system. Conventional operating systems control and schedule computer processes for execution, perform memory management, provide file system, networking, I/O services, and provide a user interface functionality, such as a graphical user interface ("GUI"), among other things.

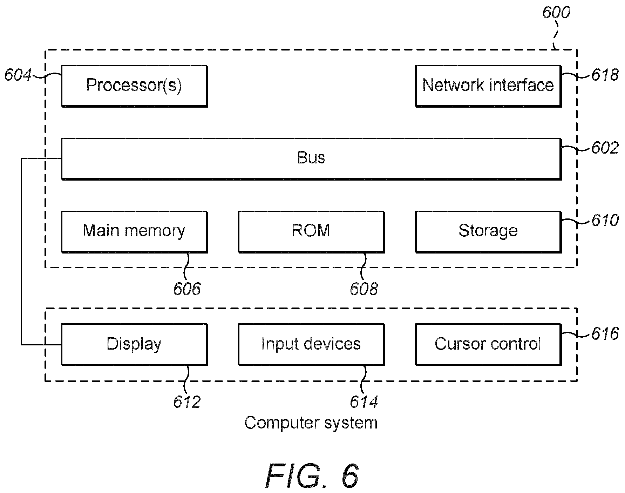

Fig. 6 is a block diagram that illustrates a computer system 600 upon which any of the embodiments of this specification may be implemented. The computer system 600 includes a bus 602 or other communication mechanism for communicating information, one or more hardware processors 604 coupled with bus 602 for processing information. Hardware processor(s) 604 may be, for example, one or more general purpose microprocessors.

The computer system 600 also includes a main memory 606, such as a random access memory (RAM), cache and/or other dynamic storage devices, coupled to bus 602 for storing information and instructions to be executed by processor 604. Main memory 606 also may be used for storing temporary variables or other intermediate information during execution of instructions to be executed by processor 604. Such instructions, when stored in storage media accessible to processor 604, render computer system 600 into a special-purpose machine that is customized to perform the operation specified in the instructions.

The computer system 600 further includes a read only memory (ROM) 608 or other static storage device coupled to bus 602 for storing static information and instructions for processor 604. A storage device 610, such as a magnetic disk, optical disk, or USB thumb drive (Flash drive), etc., is provided and coupled to bus 602 for storing information and instructions.

The computer system 600 may be coupled via bus 602 to a display 612, such as a cathode ray tube (CRT) or LCD display (or touch screen), for displaying information to a computer user. An input device 614, including alphanumeric and other keys, is coupled to bus 602 for communicating information and command selections to processor 604. Another type of user input device is cursor control 616, such as mouse, a trackball, or cursor directions keys for communicating direction information and command selections to processor 604 and for controlling cursor movement on display 612. This input device typically has two degrees of freedom in two axes, a first axis (e.g., x) and a second axis (e.g., y), that allows the device to specify positions in a plane. In some embodiments, a same direction information and command selections as cursor control may be implemented via receiving touches on a touch screen without a cursor.

The computer system 600 may include a user interface module to implement a GUI that may be stored in a mass storage device as executable software codes that are executed by the computing device(s). This and other modules may include, by way of example, components, such as software components, object-oriented software components, class components and task components, processes, functions, attributes, procedures, subroutines, segments of program code, drivers, firmware, microcode, circuitry, data, databases, data structures, tables, arrays, and variables.

In general, the word "module" as used herein, refers to logic embodied in hardware or firmware, or to a collection of software instructions, possibly having entry and exit points, written in a programming language, such as, for example, Java, C or C++. A software module may be compiled and linked into an executable program, installed in a dynamic link library, or may be written in an interpreted programming language such as, for example, BASIC, Perl, or Python. It will be appreciated that software modules may be callable from other modules or from themselves, and/or may be invoked in response to detected events or interrupts. Software modules configured for execution on computing devices may be provided on a computer readable medium, such as a compact disc, digital video disc, flash drive, magnetic disc, or any other tangible medium, or as a digital download (and may be originally stored in a compressed or installable format that requires installation, decompression or decryption prior to execution). Such software code may be stored, partially or fully, on a memory device of the executing computing device, for execution by the computing device. Software instructions may be embedded in firmware, such as an EPROM.

It will be further appreciated that hardware modules may be comprised of connected logic units, such as gates and flip-flops, and/or may be comprised of programmable units, such as programmable gate arrays or processors. The modules or computing device functionality described herein are preferably implemented as software modules, but may be represented in hardware or firmware. Generally, the modules described herein refer to logical modules that may be combined with other modules or divided into sub-modules despite their physical organization or storage.

The computer system 600 may implement the techniques described herein using customized hard-wired logic, one or more ASIC or FPGAs, firmware and/or program logic which in combination with the computer system causes or programs computer system 600 to be a special-purpose machine. According to one embodiment, the techniques herein are performed by computer system 600 in response to processor(s) 604 executing one or more sequences of one or more instructions contained in main memory 606. Such instructions may be read into main memory 606 from another storage medium, such as storage device 610. Execution of the sequences of instructions contained in main memory 606 causes processor(s) 604 to perform the process steps described herein. In alternative embodiments, hard-wired circuitry may be used in place of or in combination with software instructions.

The term "non-transitory media" and similar terms, as used herein refers to any media that store data and/or instructions that cause a machine to operate in specific fashion. Such non-transitory media may comprise non-volatile media and/or volatile media. Non-volatile media includes, for example, optical or magnetic disks, such as storage device 610. Volatile media includes dynamic memory, such as main memory 606. Common forms of non-transitory media include, for example, a floppy disk, a flexible disk, hard disk, solid state drive, magnetic tape, or any other magnetic data storage medium, a CD-ROM, any other optical storage medium, any physical medium with patterns of holes, a RAM, a PROM, and EPROM, a FLASH-EPROM, NVRAM, any other memory chip or cartridge, and networked versions of a same.

Non-transitory media is distinct from but may be used in conjunction with transmission media. Transmission media participates in transferring information between non-transitory media. For example, transmission media includes coaxial cables, copper wire and fiber optics, including the wires that comprise bus 602. Transmission media can also take the form of acoustic or light waves, such as those generated during radio-wave and infra-red data communications.

Various forms of media may be involved in carrying one or more sequences of one or more instructions to processor 604 for execution. For example, the instructions can initially be carried on a magnetic disk or solid state drive of a remote computer. The remote computer may load the instructions into its dynamic memory and send the instructions over a telephone line using a modem. A modem local to computer system 600 can receive the data on the telephone line and use an infra-red transmitter to convert the data to an infra-red signal. An infra-red detector can receive the data carried in the infra-red signal and appropriate circuitry can place the data on bus 602. Bus 602 carries the data to main memory 606, from which processor 604 retrieves and executes the instructions. The instructions received by main memory 606 may optionally be stored on storage device 610 either before or after execution by processor 604.

The computer system 600 also includes a communication interface 618 coupled to bus 602 via which encoded image data or encoded video data may be received. Communication interface 618 provides a two-way data communication coupling to one or more network links that are connected to one or more local networks. For example, communication interface 618 may be an integrated services digital network (ISDN) card, cable modem, satellite modem, or a modem to provide a data communication connection to a corresponding type of telephone line. As another example, communication interface 618 may be a local area network (LAN) card to provide a data communication connection to a compatible LAN (or WAN component to communicated with a WAN). Wireless links may also be implemented. In any such implementation, communication interface 618 sends and receives electrical, electromagnetic or optical signal that carry digital data streams representing various types of information.

A network link typically provides data communication through one or more networks to other data devices. For example, a network link may provide a connection through local network to a host computer or to data equipment operated by an Internet Service Provider (ISP). The ISP in turn provides data communication services through the world wide packet data communication network now commonly referred to as the "Internet". Local network and Internet both use electrical, electromagnetic or optical signals that carry digital data streams. The signals through the various networks and the signals on network link and through communication interface 618, which carry the digital data to and from computer system 600, are example forms of transmission media.

The computer system 600 can send messages and receive data, including program code, through the network(s), network link and communication interface 618. In the Internet example, a server might transmit a requested code for an application program through the Internet, the ISP, the local network and the communication interface 618. The received code may be executed by processor 604 as it is received, and/or stored in storage device 610, or other non-volatile storage for later execution.

Each of the processes, methods, and algorithms described in the preceding sections may be embodied in, and fully or partially automated by, code modules executed by one or more computer systems or computer processors comprising computer hardware. The processes and algorithms may be implemented partially or wholly in application-specific circuitry.

The various features and processes described above may be used independently of one another, or may be combined in various ways. All possible combination and subcombinations are intended to fall within the scope of this disclosure. In addition, certain method or process blocks may be omitted in some implementations. The methods and processes described herein are also not limited to any particular sequence, and the blocks or states relating thereto can be performed in other sequences that are appropriate. For example, described blocks or states may be performed in an order other than that specifically disclosed, or multiple blocks or states may be combined in a single block or state. The example blocks or states may be performed in serial, in parallel, or in some other manner. Blocks or states may be added to or removed from the disclosed example embodiments. The example systems and components described herein may be configured differently than described. For example, elements may be added to, removed from, or rearranged compared to the disclosed example embodiments.

Conditional language, such as, among others, "can", "could", "might", or "may" unless specifically stated otherwise, or otherwise understood within the context as used, is generally intended to convey that certain embodiments include, while other embodiments do not include, certain features, elements and/or steps. Thus, such conditional language is not generally intended to imply that features, elements and/or steps are in a way required for one or more embodiments or that one or more embodiments necessarily include logic for deciding, with or without user input or prompting, whether these features, elements and/or steps are included or are to be performed in any particular embodiment.

Any process descriptions, elements, or blocks in the flow diagrams described herein and/or depicted in the attached figures should be understood as potentially representing modules, segments, or portions of code which include one or more executable instructions for implementing specific logical functions or steps in the process. Alternate implementations are included within the scope of the embodiments described herein in which elements or functions may be deleted, executed out of order from that shown or discussed, including substantially concurrently or in reverse order, depending on the functionality involved, as would be understood by those skilled in the art.

It should be emphasized that many variations and modification may be made to the above-describe embodiments, the elements of which are to be understood as being among other acceptable examples. All such modifications and variations are intended to be included herein within the scope of this disclosure. The foregoing description details certain embodiments of the disclosure. It will be appreciated, however, that no matter how detailed the foregoing appears in text, the concept can be practiced in many ways. As is also stated above, it should be noted that the use of particular terminology when describing certain features or aspects of the disclosure should not be taken to imply that the terminology is being re-defined herein to be restricted to including any specific characteristics of the features or aspects of the disclosure with which that terminology is associated. The scope of the protection should therefore be construed in accordance with the appended claims and equivalents thereof.

## Claims

1. A method of providing feedback to a machine learning model, the method comprising:
allowing a user to observe an output of a trained machine learning model;
allowing the user to input feedback to the machine learning model based on the output, wherein the feedback is on at least one of a model level or on a training dataset level; and
incorporating the feedback into the machine learning model to improve the machine learning model,
wherein the method is performed using one or more processors.

2. The method of claim 1, wherein the feedback on the model level comprises qualitative feedback on the machine learning model

3. The method of claim 2, wherein the feedback on the model relates to features and/or hyperparameters of the machine learning model, the method further comprising
retraining the machine learning model; and
looping back to the step of allowing a user to observe an output of a trained machine learning model to iteratively improve the machine learning model.

4. The method of claim 2 or claim 3, wherein the qualitative feedback is reviewed by a user and manually incorporated into the machine learning model.

5. The method of claim 1, wherein the feedback on the dataset level comprises quantitative feedback on the output of the machine learning model and wherein incorporating the feedback into the machine learning model comprises:
writing back the feedback in a write-back dataset; and
merging the write-back dataset with the training dataset;
the method further comprising
retraining the machine learning model with the training dataset; and looping back to the step of allowing a user to observe an output of a trained machine learning model to iteratively improve the machine learning model.

6. The method of claim 5, wherein the user is allowed to flag the prediction output of the machine learning model as a false positive for a binary classification problem, and/or wherein when the prediction output is a predicted time period for an event the user is allowed to indicate how long the event took in reality, and optionally wherein the event is a maintenance task

7. The method of any preceding claim, wherein the incorporating of the feedback is performed as write-backs.

8. The method of any preceding claim, wherein the quantitative feedback is automatically incorporated into the machine learning model.

9. The method of any preceding claim, wherein the feedback captures accumulated experience and know-how of subject-matter experts over time.

10. The method of any preceding claim, wherein the feedback is bias monitored and/or wherein the training dataset is bias monitored.

11. The method of any preceding claim, wherein the feedback on the model level and the feedback on the training dataset level are input to the machine learning model via one common graphical user interface.

12. The method of any preceding claim, wherein the method is used in a system to predict a variable based on medical images, the user is a clinician and allowing the user to input feedback comprises selecting an area of the image by the clinician, passing the selected area of the image to the machine learning model for a preliminary prognosis, evaluating the output of the model by the clinician and inputting the evaluation as feedback,

13. The method of claim 12, wherein the feedback is quantitative feedback and comprises medical images annotated by the clinician, and/or wherein the variable is a survival rate of lung cancer patients and the medical images are images showing human lungs or parts thereof.

14. Computer instructions which, when executed by one or more processors, cause the one or more processors to perform the method of any one of the preceding claims.

15. A computer system comprising:
one or more processors; and
one or more computer-readable storage media comprising computer executable instructions which when executed by the one or more processors cause the one or more processors to perform the method of any one of the claims 1 to 13.
